# EUROPEAN PATENT APPLICATION

(11) **EP 2 348 267 A2**
(43) Date of publication of application: **27.07.2011**
(21) Application number: 11000223.5
(22) Date of filing: 13.01.2011
(51) Int. Cl.: F25D 19/00, G01R 33/3815, H01F 6/04

(54) **Methods for recovering helium**

(30) Priority: 20.01.2010 US 690517
(71) Applicant: Linde AG, 80331 München (DE)
(72) Inventor: Glajchen, Mark, Livingston NJ 07039 (US); Fiorino, Gary, Martinsville NJ 08836 (US); Pivonka, Thomas, Albert KS 67511 (US)
(74) Representative: Zahn, Christoph

(57) **Abstract**

A method for providing cooling to the superconducting coils of a magnet with cryogenic helium gas at a helium production facility is disclosed. The cryogenic helium gas is fed to the magnet where the helium provides cooling. Cryogenic liquid helium provides additional cooling and fills the helium reservoir. Methods are also provided for recovering vaporized helium from the filling of a magnet and when the magnet is undergoing energization at the helium production facility.

## Description

### BACKGROUND OF THE INVENTION

The invention provides for methods for integrating a helium cool down process and subsequent magnet energization process for superconducting magnets with a helium recovery process at a helium production facility. More particularly, the invention provides for using cryogenic helium gas to cool down the superconducting coils of the magnet, the subsequent filling of the magnet helium cryostat (reservoir) with liquid helium, the recovery of the associated helium gas from these activities, recovery of helium during normal boil off while the magnet is ramping and the recovery of the helium gas released as a result of a quench that may occur during the energizing (ramping) of the magnet.

Magnetic resonance imaging (MRI) and nuclear magnetic resonance (NMR) systems employing superconductive or other types of magnets are employed in fields such as medical diagnostics. The superconductive magnets comprise a coil assembly having a main coil which is at least partially immersed in liquid helium contained in a helium reservoir. The reservoir is typically surrounded by dual thermal shields which in turn are surrounded by a vacuum enclosure. Nb-Ti superconductive coils typically operate at a temperature of approximately 4 Kelvin, and Nb-Sn superconductive coils typically operate at a temperature of approximately 10 Kelvin. When the coil assembly is cooled to such a temperature it becomes superconductive and the magnet field strength is maintained without significant further energy input. A necessity for the operation of a superconducting magnet is the presence of a coolant. This coolant is typically liquid helium which can achieve the low temperatures necessary to allow the material of the magnet coil to reach a superconductive state. This need for low temperatures necessitates that the reservoir in the magnet must be filled with a sufficient amount of liquid helium at a cold enough temperature to allow the magnet coils to become superconducting.

Typically the cooling and subsequent filling of the magnet assembly with helium is first done at the magnet production or manufacturing facility and then sometimes repeated at the customer site, for example at a medical diagnostics facility. Helium will warm in the process of providing cooling to a material and some helium will enter the gaseous phase. Consequently, the magnets need to be refilled with helium regularly during the initial cooling operation and periodically during the ongoing operation. The filling or refilling operation must be carefully performed as contact with the helium is dangerous and improper handling of helium can be wasteful. The magnet must be filled with liquid helium before the superconducting coils can be energized.

These filling and refilling operations have their drawbacks as the helium must be transported to the manufacturing or customer site, adding to the cost of the operation when filling the space around the superconducting coils. In addition loses of helium due to volatilization are a factor to consider in transporting helium extended distances. Additionally, specially designed equipment may need to be fabricated and installed at a consuming location to provide the necessary helium transportation, filling and refilling. Often times in order to reduce helium costs liquid nitrogen is used as the initial coolant down to approximately 80K. This nitrogen must then be removed from the cryostat to prevent freezing when liquid helium is introduced.

The invention seeks to overcome these difficulties by providing cryogenic helium gas cooling, liquid helium fill and helium gas recovery systems at existing helium production facilities. Reduced capital costs, greatly enhanced helium recovery and elimination of nitrogen as a coolant are benefits realized by the invention. Helium vented from the MRI units during cool-down and testing can be recovered rather than simply lost. Special filling equipment, once fabricated, has sustained usage at the production facility. The invention further provides the use of onsite

helium liquefier technology as a source of cryogenic helium gas for use in cooling down the superconductive coils of a magnet from ambient temperatures.

### SUMMARY OF THE INVENTION

The invention provides for a method for cooling the superconducting coils of a magnet with cryogenic helium gas at a helium production facility comprising directing cryogenic gaseous helium from one of the stages of an onsite helium liquefier to the magnet and flowing it through the cryostat of the unit. Once the helium gas has provided cooling to the cryostat (reservoir) and coils of the magnet the product is recovered to the liquefier for reprocessing.

The invention also provides for a method of recovery of the vaporized product that results from the filling process back to the helium production plant during the filling of a magnet with liquid helium.

The invention further provides for a method of recovery at a helium production facility of cryogenic gaseous helium released during the energizing (ramping) process undertaken for a magnet and any resultant quenches that may occur from this process.

The magnet that is cooled, filled with the liquid helium and energized may be later used in equipment, such as an MRI or NMR, where a superconducting magnet is employed.

### BRIEF DESCRIPTION OF THE DRAWINGS

Fig. 1 is a schematic of a helium cooling and filling operation taking place at a helium production facility where the cryogenic helium gas is recovered back to the production plant liquefiers.

Fig. 2 is a schematic of a method of recovery of cryogenic gaseous helium released during the energizing (ramping) process undertaken for a magnet and any resultant quenches that may occur from this process.

### DETAILED DESCRIPTION OF THE INVENTION

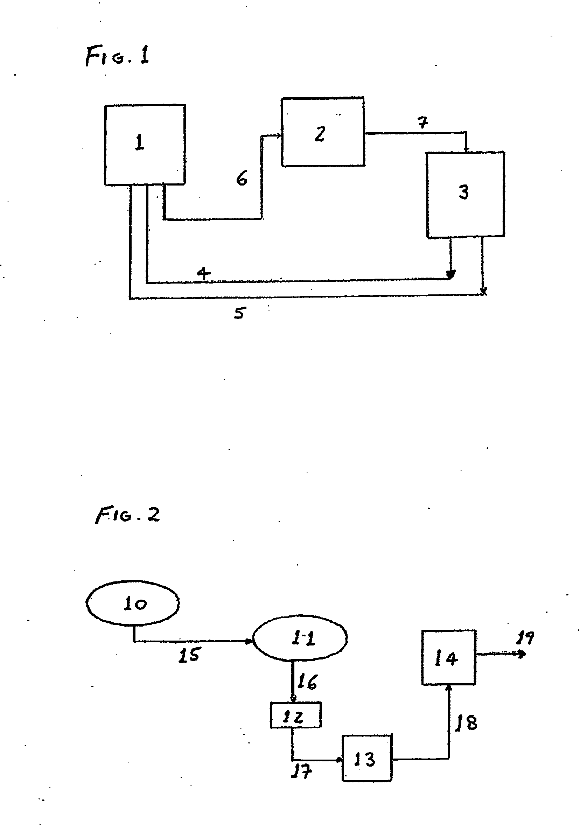

The invention will now be described with respect to Figure 1. For purposes of illustration, the following details briefly what each item in Figure 1 represents:

Item 1 is the magnet which is being cooled and subsequently filled with liquid helium;

Item 2 is the helium purifier;

Item 3 is the helium liquefier which is producing liquid helium as well as cryogenic helium gas;

Item 4 is cryogenic helium gas supply line;

Item 5 is liquid helium supply line;

Item 6 is the pipe connecting to the magnet for helium recovery;

Item 7 is the pipe connecting the helium purifier and the helium liquefier.

The cooling is commenced on a warm magnet 1 by connecting it to the supply line 4 which is in turn connected to the pure helium production stream of the helium liquefier 3. This pure helium production stream is a direct stream from a helium production facility. This stream can be at various temperatures depending on the liquefier 3 design and the point in the process where it is connected. It is suggested that a typical temperature is approx 20 K. Once this gas supply line is connected a second line 6 is connected to the magnet 1 in order to recover the helium gas back to the helium purifier 2. Once the line 6 is connected, cryogenic gas is permitted to flow into the magnet 1 to cool down the super conducting coils and support structure. This process is continued until the magnet temperature is stable.

Once the magnet temperature is stable, the helium gas supply line 4 is disconnected and the liquid helium supply line 5 from the liquefier 3 is connected and liquid helium is permitted to flow into the magnet 1 to continue the cooling process. The supply of liquid helium is permitted to continue until the helium reservoir is filled with liquid helium to the designated design maximum.

Throughout the supply of liquid helium, any helium gas present that is a result of vaporization within the magnet 1 is recovered through the line 6 that is connected to the helium purifier 2. The purified helium can be fed through line 7 to the helium liquefier 3. Once the magnet 1 is filled to the designated design maximum, the flow of liquid helium is terminated and lines 5 and 6 are disconnected.

Figure 2 is a schematic of a magnet during the energizing (ramping) process. For purposes of illustration, the following details briefly what each item in Figure 2 represents:

Item 10 is the magnet filled with liquid helium which is being energized;

Item 11 is the helium gas collection system;

Item 12 is the helium compressor;

Item 13 is the helium purifier;

Item 14 is the helium liquefier;

Item 15 is the pipe connecting to the magnet for helium recovery;

Item 16 is the pipe connecting the helium gas bag and the compressor;

Item 17 is the pipe connecting the compressor and the helium purifier;

Item 18 is the pipe connecting the helium purifier and the helium liquefier;

Item 19 is the pipe leaving the helium liquefier and directed towards storage tanks.

Prior to the commencement of the energizing (ramping) activities on a cold magnet 10 a gas collection pipe 15 is connected to the magnet 10. The intention of this line 15 is to facilitate the transfer of any helium produced as a result of the energizing process. This gas could be the product of ambient heat in-leak into the helium reservoir of the magnet 10, the gas formed due to the ramping of the superconducting coils producing heat which is dissipated into the helium reservoir or where the temporary change in the coils from a superconductive to non-superconductive state results in the dissipation of the entirety of the energy stored within the coils into the helium within the reservoir (i.e. a quench).

In the case of a quench the volume of gas released into the gas collection line and subsequently directed to the helium gas collection system 11 is large and occurs in a small amount of time, typically in under one minute, therefore it is important to size the collection line 15 and helium gas collection system 11 appropriately to handle this volume of gas in a small amount of time. A helium gas bag may be used as the helium gas collection system 11. From the helium gas collection system 11 the helium is directed through line 16 to a compressor 12 which in turn boost the pressure sufficiently to introduce the helium gas through line 17 back into the helium purifier 13 and through line 18 into helium liquefier 14, and ultimately through line 19 into storage tanks (not shown) associated with a helium production facility. The helium can then be employed at the helium production facility as the operator so desires.

While this invention has been described with respect to particular embodiments thereof, it is apparent that numerous other forms and modifications of the invention will be obvious to those skilled in the art. The appended claims in this invention generally should be construed to cover all such obvious forms and modifications which are within the true spirit and scope of the invention.

## Claims

1. A method for providing cooling to superconducting coils of a magnet with cryogenic helium gas at a helium production facility comprising feeding cryogenic gaseous helium from an onsite helium liquefier to said magnet and flowing said gaseous helium through a cryostat of said magnet.

2. The method as claimed in claim 1 wherein the temperature of said gaseous helium is 20 K.

3. The method as claimed in claim 1 or 2 wherein said helium is fed from said onsite helium liquefier until the magnet temperature is stable.

4. The method as claimed in any one of claims 1 to 3 wherein said magnet filled with liquid helium is used in a device selected from the group consisting of magnetic resonance imaging and nuclear magnetic resonance.

5. The method as claimed in claim 3 or 4 further comprising filling said magnet from said onsite helium liquefier until said cryostat is filled to its designated design maximum.

6. The method as claimed in any one of claims 1 to 5 further comprising recovering said cryogenic helium gas from said magnet.

7. The method as claimed in any one of claims 1 to 6 further comprising recovering vaporized helium from filling of a magnet with liquid helium at a helium production facility.

8. The method as claimed in any one of claims 1 to 7 wherein said vaporized helium is fed to a helium purifier.

9. The method as claimed in any one of claims 1 to 8 wherein said vaporized and purified helium is fed to a helium liquefier.

10. The method as claimed in any one of claims 1 to 9 wherein said helium liquefier supplies liquid helium to said magnet.

11. The method as claimed in any one of claims 1 to 10 further comprising recovering vaporized helium from a magnet undergoing energizing at a helium production facility.

12. The method as claimed in any one of claims 1 to 11 wherein said vaporized helium is the result of a process selected from the group consisting of superconducting coils producing heat into liquid helium and dissipation of energy from superconducting coils into liquid helium.

13. The method as claimed in any one of claims 1 to 12 where said vaporized helium is first recovered into a gas collection pipe.

14. The method as claimed in any one of claims 1 to 13 wherein said gas collection pipe is in fluid communication with a helium gas collection system.

15. The method as claimed in claim 14 wherein said helium gas collection system is a helium gas bag.
